# EUROPEAN PATENT APPLICATION

(11) **EP 4 831 166 A1**
(43) Date of publication of application: **28.10.2026**
(21) Application number: 24906197.9
(22) Date of filing: 11.12.2024
(51) Int. Cl.: C07D 337/12, C07D 337/00, C07D 337/08, C07C 205/06, C07C 47/00, C07C 211/00, A61K 31/38, A61P 43/00

(54) **METHOD FOR PREPARING INTERMEDIATE COMPOUND OF DRUG MARALIXIBAT AND USE THEREOF**

(30) Priority: 18.12.2023 CN 202311737549
(71) Applicant: Zhejiang Ausun Pharmaceutical Co., Ltd., Taizhou, Zhejiang 317016 (CN)
(72) Inventor: ZHU, Baoyun, Zhejiang 317016 (CN); HUANG, Qiuhong, Zhejiang 317016 (CN); WANG, Kuankuan, Zhejiang 317016 (CN); ZHANG, Lirong, Zhejiang 317016 (CN); DAI, Chunguang, Zhejiang 317016 (CN); PU, Xuecan, Zhejiang 317016 (CN); ZHENG, Zhiguo, Zhejiang 317016 (CN)
(74) Representative: Hoffmann Eitle
(86) International application number: PCT/CN2024/138385
(87) International publication number: WO 2025/130714

(57) **Abstract**

The present invention relates to a method for preparing a compound of formula I as an important intermediate for the drug Maralixibat, and use thereof. The compound of formula I can be synthesized by using Compound 5 or Compound 2 as a starting compound, wherein the Compound 2 is reacted with Compound S5, then reduced twice to obtain the Compound 5, the Compound 5 is then sequentially subjected to amino protection, chiral catalytic sulfur atom oxidation, terminal hydroxyl oxidation, cyclization, further sulfur atom oxidation, and reductive amination to obtain the compound of formula I, wherein R¹ and R² are as defined herein. The present invention provides novel methods for synthesizing the compound of formula I with different starting compounds. The methods of the present invention have advantages such as simple steps, mild reaction conditions, high yield, high purity, etc., and are suitable for industrial-scale production. In addition, the resultant compound of formula I and the intermediate compounds involved in the synthesis process can be applied to the preparation of the drug Maralixibat.

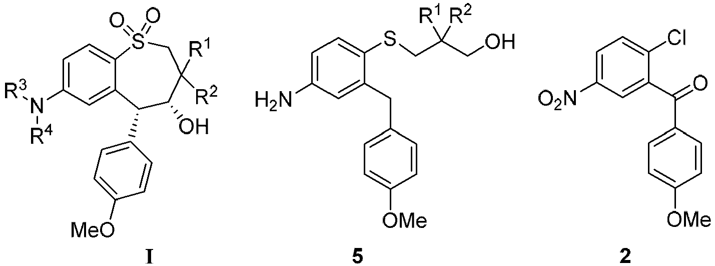

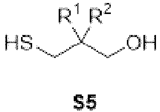

## Description

### Cross reference to related application

The present application claims the priority of Chinese patent application No. 202311737549.4 filed to the Chinese Patent Office on December 18, 2023, which is incorporated herein by reference.

### Technical Field

The present invention relates to the field of pharmaceutical chemistry, and particularly to a method for preparing an intermediate compound of Maralixibat which is a drug for treating cholestatic pruritus in patients with Alageri syndrome, and use thereof.

### Background Art

Alageri syndrome (ALGS) is a rare hereditary disease characterized by abnormal stenosis, malformation, and number reduction of bile ducts, leading to the accumulation of bile within the liver and thus causing progressive liver diseases. In patients with Alageri syndrome, multiple organ systems may be affected by gene mutations, including the liver, heart, kidneys and central nervous system.

The accumulation of bile acids renders the liver unable to function normally, and thus unable to clear wastes from the blood. It is reported that 60% to 75% of the patients with Alageri syndrome undergo liver transplantation before reaching adulthood. Signs and symptoms resulting from liver damage associated with Alageri syndrome include jaundice, xanthoma, and pruritus. The pruritus experienced by the patients with Alageri syndrome is the most severe in any chronic liver disease, and symptoms typically appears in the most affected children before the age of three.

In September 2021, Maralixibat oral liquid was approved for marketing in the United States, becoming the first and only U.S. FDA-approved drug for the treatment of cholestatic pruritus in children aged one year or above with Alageri syndrome. In June 2023, Maralixibat oral liquid became the first drug approved in China for the treatment of cholestatic pruritus in patients with Alageri syndrome. The chemical structure of Maralixibat is as shown below:

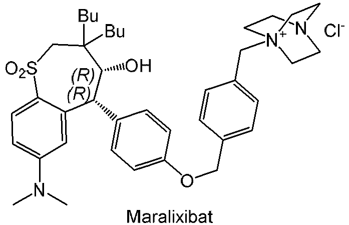

CN1284953A discloses a method for preparing an intermediate compound of formula Ia for Maralixibat, in which the product is obtained from p-fluorophenol as the starting material by sequentially subjecting to four substitution reactions, two oxidation reactions, a cyclization reaction, and a final oxidation reaction: 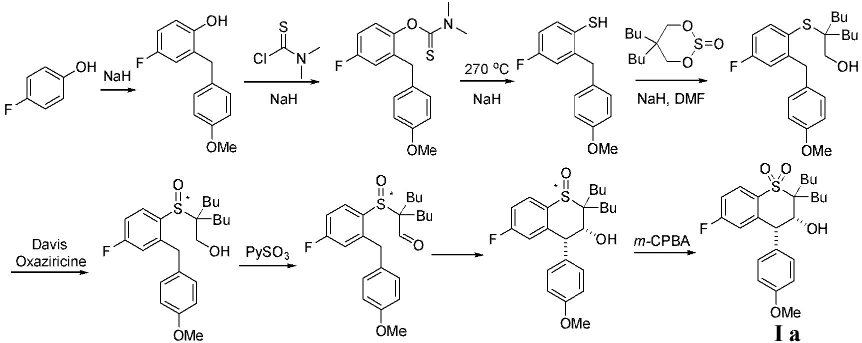 However, the multiple reaction steps of this method require the use of hazardous chemical reagents such as sodium hydride (NaH), and at least one step therein requires a reaction temperature as high as 270°C, entailing harsh reaction conditions. Furthermore, the step involving the conversion of thiophenol into a chiral sulfoxide compound requires the expensive chiral ligand Davis Oxiziricine, and the multiple reaction steps require purification via column chromatography, so that the preparation process is complex and costly, and thus is unsuitable for industrial-scale production.

WO2001068637A2 discloses a method for preparing an intermediate compound of formula Ib for Maralixibat, of which the Reaction Scheme is as follows: 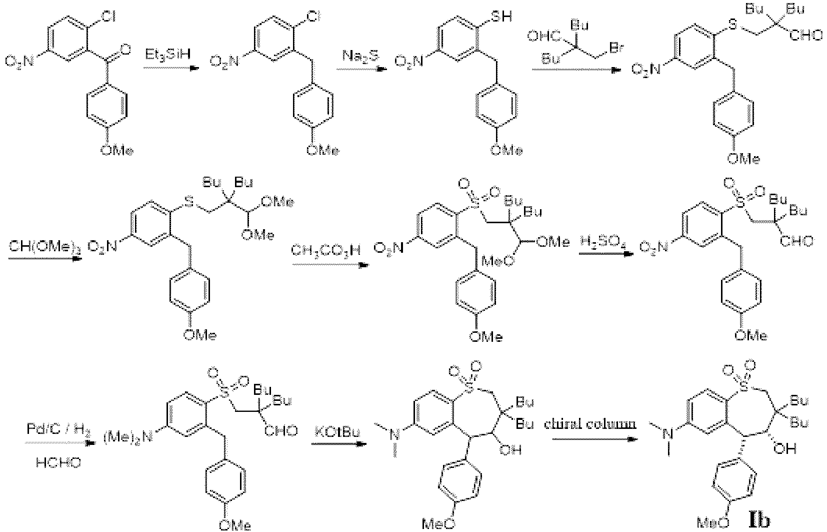 However, this method requires redundant steps involving in the protection and deprotection for the aldehyde group, as well as post-treatment steps such as separation via chiral column to obtain the target compound, all of which are unfavorable for industrial-scale production.

Therefore, there is a need in the art to develop and design novel methods for preparing key intermediate compounds for Maralixibat.

### Summary of Invention

In order to overcome the above shortcomings of the prior art, the present invention develops and designs a novel method for preparing a compound of formula I, which is an important intermediate for Maralixibat. By reducing or eliminating the use of expensive and specific chiral ligands such as Davis Oxiziricine, as well as reducing the treatment steps via column chromatography, and eliminating the step via chiral column separation, the present method enables the industrial-scale production of a compound of formula I in a more economical, simpler and more cost-effective way.

To this end, in one aspect, the present invention provides a method for preparing a compound of formula I, comprising: 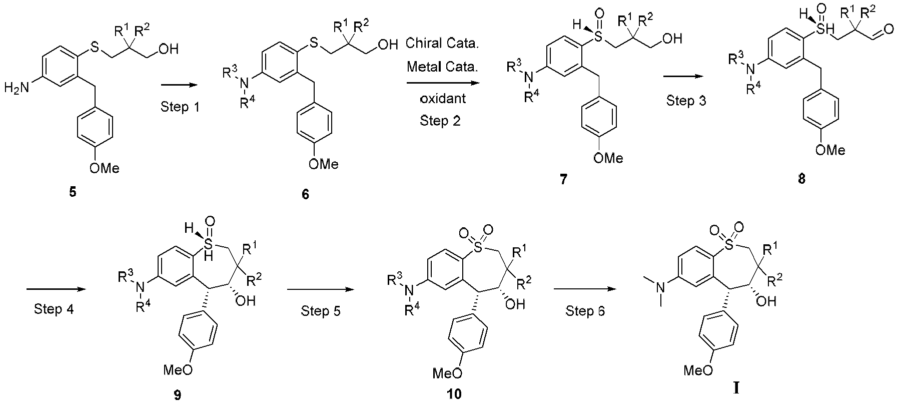
Step 1: reacting Compound 5 with a protecting reagent bearing amino-protecting groups R³ and R⁴ to obtain Compound 6;
Step 2: oxidizing the resultant Compound 6 with a first oxidant in the presence of a chiral ligand and a metal catalyst to obtain Compound 7;
Step 3: reacting the resultant Compound 7 with a second oxidant to obtain Compound 8;
Step 4: cyclizing the resultant Compound 8 under an alkaline condition to obtain Compound 9;
Step 5: reacting the resultant Compound 9 with a third oxidant to obtain Compound 10; and
Step 6: reacting the resultant Compound 10 with a methylamination reagent in the presence of a first reductant to obtain the compound of formula I,
wherein R¹ and R² are independently selected from H and C₁₋₁₀ alkyl, and R³ and R⁴ are amino-protecting groups independently selected from H, benzyl, 4-methoxybenzyl, benzylformyl, tert-butoxycarbonyl, formyl, acetyl, trifluoroacetyl and 9-fluorenylmethoxycarbonyl, provided that both R³ and R⁴ are not simultaneously H.

In preferred embodiments, R¹ and R² are independently selected from H, methyl, ethyl, n-propyl, isopropyl, n-butyl, isobutyl, n-pentyl, isopentyl, n-hexyl, n-heptyl, n-octyl, n-nonyl and n-decyl.

In preferred embodiments, R¹ and R² are independently selected from H, methyl, ethyl, n-propyl and n-butyl.

In preferred embodiments, the chiral ligand used in Step 2 is one or more selected from

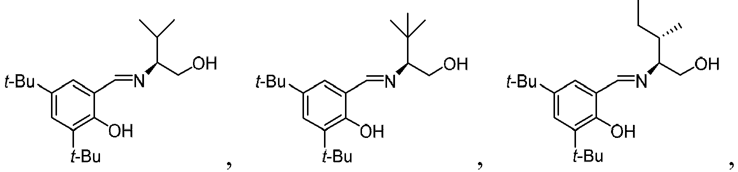

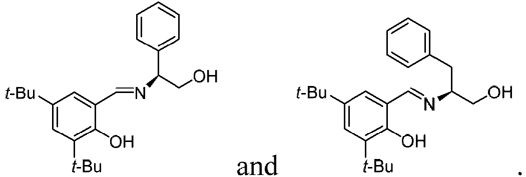

In preferred embodiments, the metal catalyst used in Step 2 is one or more selected from PdCl₂, NiCl₂, Pd(OH)₂, Ni(acac)₂, RhCl₂ and CrCl₂.

In preferred embodiments, the first oxidant used in Step 2 is one or more selected from H₂O₂, 3-phenyl-2-phenylsulfonyl-1,2-oxaziridine and benzoyl peroxide.

In preferred embodiments, the second oxidant used in Step 3 is one or more selected from potassium permanganate, manganese dioxide and sodium hypochlorite.

In preferred embodiments, a base used for the alkaline condition in Step 4 is one or more selected from sodium methoxide, sodium ethoxide, sodium tert-butoxide, potassium tert-butoxide, sodium hydroxide, potassium hydroxide, cesium hydroxide, sodium carbonate, potassium carbonate, potassium bicarbonate, NaH, KH, lithium diisopropylamide, lithium hexamethyldisilamide and sodium hexamethyldisilamide.

In preferred embodiments, the third oxidant used in Step 5 is one or more selected from peroxyacetic acid, sodium periodate and m-chloroperoxybenzoic acid.

In preferred embodiments, the first reductant used in Step 6 is one or more selected from Pd/C with H₂, Raney nickel with H₂, or zinc powder with H₂.

In preferred embodiments, the methylamination reagent used in Step 6 is one or more selected from formaldehyde, trioxymethylene, and paraformaldehyde.

In preferred embodiments, the Compound 5 used in Step 1 is prepared by a method comprising the steps of: 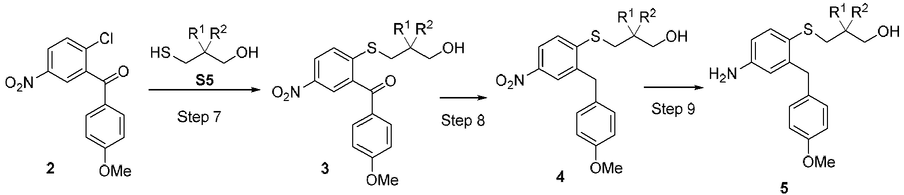
Step 7: reacting Compound 2 with Compound S5 under an alkaline condition to obtain Compound 3;
Step 8: reducing the Compound 3 with a second reductant to obtain Compound 4; and
Step 9: reducing the Compound 4 with a third reductant to obtain the Compound 5;
wherein R¹ and R² are as described above.

In preferred embodiments, a base used for the alkaline condition in Step 7 is one or more selected from potassium carbonate, sodium carbonate, potassium phosphate, potassium bicarbonate and sodium bicarbonate.

In preferred embodiments, the second reductant used in Step 8 is one or more selected from sodium borohydride, potassium borohydride, borane, trimethylsilane, triethylsilane and tripropylsilane.

In preferred embodiments, the third reductant used in Step 9 is one or more selected from Pd/C/H₂, Raney nickel/H₂, iron/hydrochloric acid, or zinc/hydrochloric acid.

In another aspect, the present invention provides intermediate compounds as shown below for preparing drug Maralixibat: 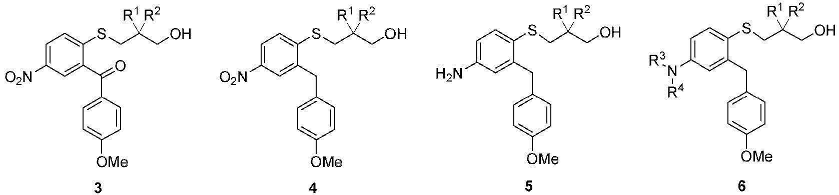 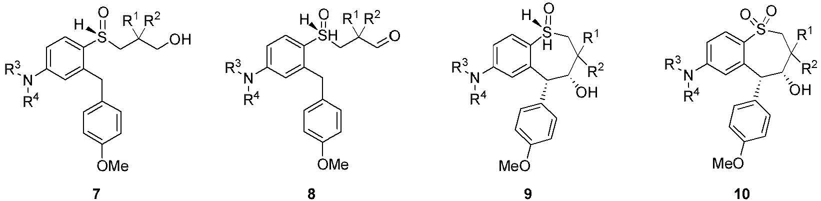 wherein R¹, R², R³ and R⁴ are as defined above.

In preferred embodiments, the intermediate compounds are selected from: 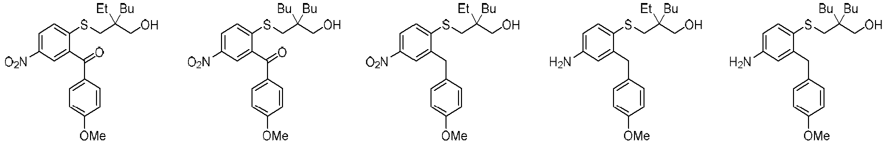 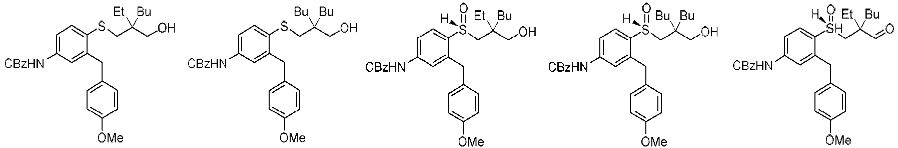 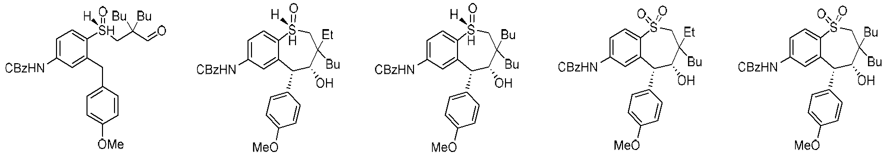 wherein Me represents methyl, Et represents ethyl, Bu represents butyl, and CBz represents benzyloxycarbonyl.

In another aspect, the present invention provides use of the compound of formula I as described above or the intermediate compounds as described above in preparing the drug Maralixibat.

The present invention provides novel methods for synthesizing a compound of formula I, as an important intermediate for the drug Maralixibat, starting with different starting compounds. The methods of the present invention have advantages such as simple steps, mild reaction conditions, high yield, high purity, etc., and are suitable for industrial-scale production. In addition, the resultant compound of formula I and the intermediate compounds involved in the synthesis processes can be applied to preparing the drug Maralixibat.

### Detailed Description

The following detailed description is provided to assist those skilled in the art to carry out and implement the present invention. However, it should be understood that this description should not be construed as limiting the present invention in any inappropriate way. In addition, the content of each of the literatures mentioned herein is also incorporated herein by reference for all purposes.

The method of the present invention uses a derivative of 3-((4-amino-2-(4-methoxy)benzyl)-phenylthio)propan-1-ol (i.e., Compound 5) as a starting compound for synthesis, wherein the Compound 5 is sequentially subjectd to amino protection, chiral catalytic sulfur atom (S) oxidation, terminal hydroxyl oxidation, cyclization, further sulfur atom (S) oxidation and reductive amination to obtain the compound of formula I, which is an important intermediate for the drug Maralixibat. Preferably, the above Compound 5 may be synthesized by using 1-chloro-2-(4-methoxybenzyl)-4-nitrobenzene (i.e., Compound 2) as a starting compound, wherein the Compound 2 is reacted with a derivative of 3-mercapto-propan-1-ol (i.e., Compound S5), and then is reduced twice to obtain the Compound 5.

Specifically, the method of the present invention may be as shown below in Reaction Schemes 1 and 2:

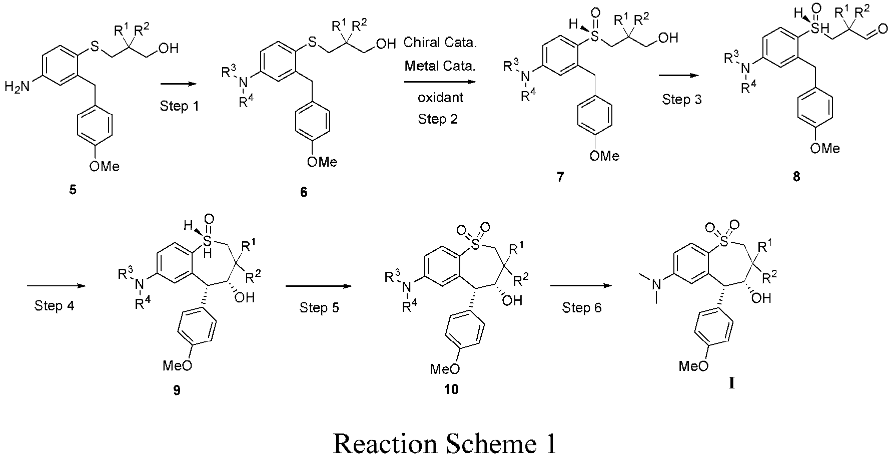

Preferably, the Compound 5 used in Step 1 is prepared by the following Reaction Scheme 2:

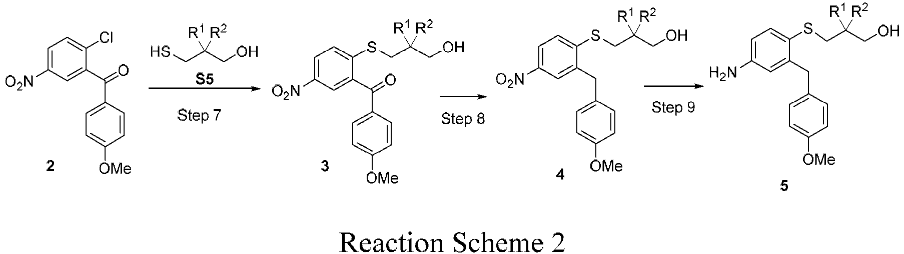

More preferably, the compound of formula I may be synthesized by using the Compound 2 as a starting compound via the following Reaction Scheme 3:

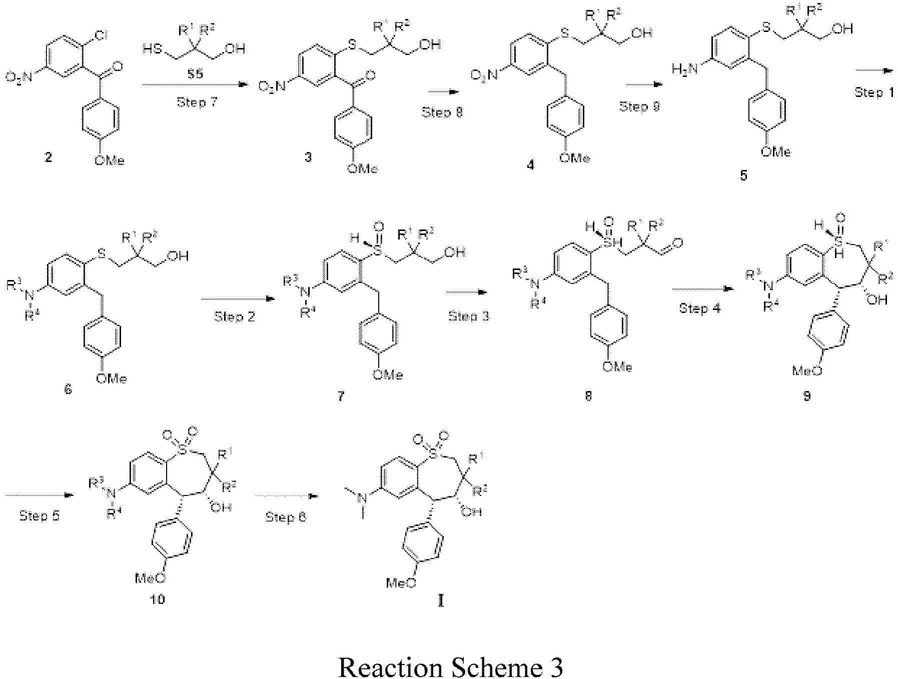

As shown above, Reaction Scheme 1 comprises the following Steps 1-6 in sequence, Reaction Scheme 2 comprises Steps 7-9 in sequence, while Reaction Scheme 3 comprises Steps 7-9 as well as subsequent Steps 1-6 in sequence.

### Step 1

Compound 5 is reacted with a protecting reagent bearing amino-protecting groups R³ and R⁴ to obtain Compound 6.

Preferably, Step 1 may be carried out as follows: Compound 5 is reacted with the protecting reagent bearing amino-protecting groups R³ and R⁴ in an organic solvent in the presence of a base at a temperature of -5 to 25°C, to replace two H atoms on the amino group of Compound 5 with the amino-protecting groups R³ and R⁴ respectively to obtain the Compound 6.

In the present invention, the groups R¹ and R² in Compound 5 may be independently selected from H and C₁₋₁₀ alkyl. For example, R¹ and R² may be independently selected from H, methyl, ethyl, n-propyl, isopropyl, n-butyl, isobutyl, n-pentyl, isopentyl, n-hexyl, n-heptyl, n-octyl, n-nonyl and n-decyl. Further, for example, R¹ and R² may be independently selected from H, methyl, ethyl, n-propyl and n-butyl.

In the present invention, the amino-protecting groups R³ and R⁴ may be amino-protecting groups independently selected from H, benzyl, 4-methoxybenzyl, benzylformyl, tert-butoxycarbonyl, formyl, acetyl, trifluoroacetyl and 9-fluorenylmethoxycarbonyl, provided that both R³ and R⁴ are not simultaneously H, that is, at least one of groups R³ and R⁴ is not H. The present inventors have found that, with the introduction of such amino-protecting groups R³ and R⁴ into the amino group of Compound 5 in Step 1, not only the amino group can be protected in the reactions of subsequent steps, but also the compound with such protection is more favorable for subsequent reactions such as selective oxidation and cyclization due to the introduction of at least one group with a larger steric hindrance or a carbonyl-containing group such as benzyloxycarbonyl onto the amino group.

In Step 1, preferably, the base used may be one or more selected from lithium hydroxide, sodium hydroxide, potassium hydroxide, lithium carbonate, sodium carbonate, potassium carbonate, sodium bicarbonate, potassium bicarbonate and potassium phosphate.

In Step 1, preferably, the organic solvent used may be one or more independently selected from tetrahydrofuran, acetonitrile, isopropanol, methanol, 1,4-dioxane, N,N-dimethylformamide, N,N-dimethylaniline, dimethyl sulfoxide, dichloromethane, chloroform, 1,2-dichloroethane, toluene, ethyl acetate and acetone.

In the present invention, more specifically, for example, Step 1 may be carried out as follows: Compound 5 is dissolved in an organic solvent, and an inorganic base is added and stirring is started. Then, the mixture is cooled down to a temperature of -5°C to 15°C, and the above protecting reagent is added dropwise. After completion of the dropwise addition, the reaction is kept at that temperature for 2 to 6 hours. After the reaction is completed, water is added and the mixture is separated into layer. The organic phase is washed with water, and then concentrated to dryness to obtain Compound 6 as an oil.

### Step 2

The resultant Compound 6 is oxidized with a first oxidant in the presence of a chiral ligand and a metal catalyst to obtain Compound 7.

Preferably, Step 2 may be carried out as follows: the resultant Compound 6 is oxidized with a first oxidant in an organic solvent in the presence of a chiral ligand and a metal catalyst at a temperature of -5°C to 20°C to obtain Compound 7.

In Step 2, preferably, the chiral ligand used may be one or more selected from:

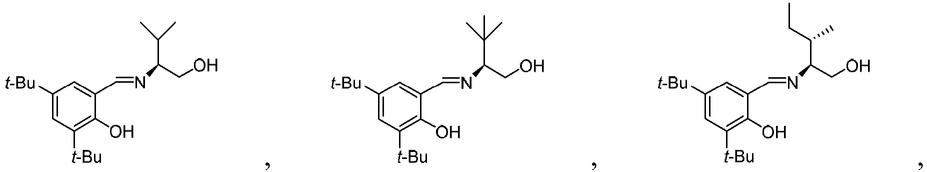

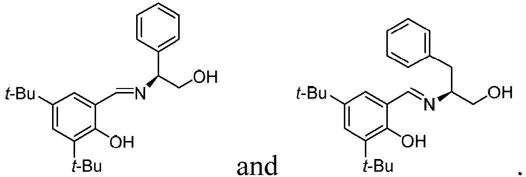

In Step 2, preferably, the metal catalyst used may be one or more selected from PdCl₂, NiCl₂, Pd(OH)₂, Ni(acac)₂, RhCl₂ and CrCl₂. Such metal catalysts are commercially available, and can not only promote the occurrence of the reaction, but also have no influence on the chirality of the sulfur center of Compound 7. In Step 2, preferably, the first oxidant used may be one or more selected from H₂O₂, 3-phenyl-2-phenylsulfonyl-1,2-oxaziridine and benzoyl peroxide. Such first oxidants are known in the art, and are commercially available.

In Step 2, preferably, in the presence of the above chiral ligand and metal catalyst, and by using the above first oxidant, oxidation may be occurred only one time on the sulfur atom (S) in Compound 6, thereby yielding the sulfur-centered chiral Compound 7 with a high enantioselectivity.

In the present invention, preferably, the organic solvent used in Step 2 may be an organic solvent independently selected from those solvents described above for Step 1.

In the present invention, more specifically, for example, Step 2 may be carried out as follows: the metal catalyst and the chiral ligand are added to an organic solvent, and after stirring for 10-30 minutes, Compound 6 is added thereto. Then, the mixture is cooled down to a temperature of -5°C to 10°C, and an organic solution of a first oxidant is slowly added dropwise. After the reaction is completed, an aqueous sodium thiosulfate solution is added to terminate the reaction, and the reaction solution is spin-dried. Then, an organic solvent and water are added, and the mixture is separated into layers. The organic phase is washed with saline, and then concentrated to dryness to obtain the Compound 7 as a solid.

### Step 3

The resultant Compound 7 is reacted with a second oxidant to obtain Compound 8.

Preferably, Step 3 may be carried out as follows: the resultant Compound 7 is oxidized with a second oxidant in an organic solvent at a temperature of 0°C to 25°C to obtain Compound 8.

In Step 3, preferably, the second oxidant used may be one or more selected from potassium permanganate, manganese dioxide and sodium hypochlorite. Such second oxidants are known in the art, and are commercially available. In Step 3, by using such a second oxidant, only the terminal hydroxyl group (OH) of the Compound 7 can be oxidized into an aldehyde group to obtain Compound 8.

In the present invention, preferably, the inorganic base used in Step 3 may be one or more selected from sodium hydroxide, potassium hydroxide, sodium carbonate, potassium carbonate, sodium bicarbonate, potassium bicarbonate and potassium phosphate.

In the present invention, preferably, the organic solvent used in Step 3 may be an organic solvent independently selected from those solvents described above for Step 1.

In the present invention, more specifically, for example, Step 3 may be carried out as follows: Compound 7 is dissolved in an organic solvent, and stirring is started. Then, the mixture is cooled down to a temperature of 0°C to 15°C, and a second oxidant is added in portions. Then, the mixture is naturally warmed up to room temperature and is stirred for 1 to 4 h. After the reaction is completed, diatomite is added for filtration. The filtrate is spin-dried to obtain a concentrate. The concentrate is dissolved in an organic solvent, and the inorganic base is added to adjust the pH to 7 to 10. The mixture is stirred at room temperature for 3 to 6 h. Then, the mixture is suction filtered to obtain a filtrate. The filtrate is washed with a saturated saline, and is separated to obtain an organic phase. Then, the filtrate is spin-dried, and the organic phase is concentrated to dryness to obtain Compound 8 as a solid.

### Step 4

The resultant Compound 8 is cyclized under an alkaline condition to obtain Compound 9.

Preferably, Step 4 may be carried out as follows: the resultant Compound 8 is cyclized in an organic solvent in the presence of a base at a temperature of 25°C to 60°C to obtain Compound 9.

In the present invention, preferably, the inorganic base used in Step 4 may be one or more selected from sodium methoxide, sodium ethoxide, sodium tert-butoxide, potassium tert-butoxide, sodium hydroxide, potassium hydroxide, cesium hydroxide, sodium carbonate, potassium carbonate, potassium bicarbonate, sodium hydride, potassium hydride, lithium diisopropylamide, lithium hexamethyldisilamide and sodium hexamethyldisilamide.

In the present invention, preferably, the inorganic acid used in Step 4 may be one or more selected from hydrochloric acid, sulfuric acid, phosphoric acid, glacial acetic acid and citric acid.

In the present invention, preferably, the organic solvent used in Step 4 may be an organic solvent independently selected from those solvents described above for Step 1.

In the present invention, more specifically, for example, Step 4 may be carried out as follows: under an inert atmosphere such as a nitrogen atmosphere, Compound 8 is dissolved in the organic solvent, and stirring is started. Then, an aqueous solution of an inorganic base is slowly added, and the mixture is heated to a temperature of 35°C to 45°C, which is stirred at that temperature for 10-14 h. After the reaction is completed, the temperature is cooled down to a temperature of -15°C to 0°C. Then, the reaction is quenched by adding water, and an inorganic acid is added dropwise to adjust the pH to 3 to 6. After that, the mixture is separated into layers. Then, the organic layer is washed once with a saturated sodium bicarbonate solution and once with a saturated saline respectively, and then is spin-dried to obtain Compound 9 as a solid.

### Step 5

The resultant Compound 9 is reacted with a third oxidant to obtain Compound 10.

Preferably, Step 5 may be carried out as follows: the resultant Compound 9 is oxidized with a third oxidant in an organic solvent at a temperature of 0°C to 25°C to obtain Compound 10.

In Step 5, preferably, the third oxidant used may be one or more selected from peroxyacetic acid, sodium periodate, and m-chloroperoxybenzoic acid. Such third oxidants are known in the art, and are commercially available. In Step 5, by using such a third oxidant, the sulfur atom (S) in Compound 9 can be further oxidized to obtain Compound 10.

In the present invention, preferably, the organic solvent used in Step 5 may be an organic solvent independently selected from those solvents described above for Step 1.

In the present invention, more specifically, for example, Step 5 may be carried out as follows: Compound 9 is dissolved in an organic solvent, and stirring is started. Then, the mixture is cooling down to a temperature of 0°C to 10°C, and an organic solution of a third oxidant is added. After the addition is completed, the mixture is warmed up to room temperature and then kept at that temperature for 1 h. After the reaction is completed, a quencher such as a saturated NaHCO₃ aqueous solution is added to terminate the reaction. The organic phase is spin-dried, and the organic solvent such as ethyl acetate is added for extraction and layer separation. The organic layer is washed with a saturated saline to obtain an organic phase. The organic phase is spin-dried, and then is subjected to column chromatography to obtain Compound 10.

### Step 6

The resultant Compound 10 is reacted with a methylamination reagent in the presence of a first reductant to obtain the compound of formula I.

Preferably, Step 6 may be carried out as follows: the resultant Compound 10 is subjected to a reductive amination in an organic solvent in the presence of a first reductant and a methylamination reagent at a temperature of 0°C to 25°C, so that both of the amino-protecting groups R³ and R⁴ are replaced with methyl, and thus the compound of formula I is obtained.

In Step 6, preferably, the first reductant used may be one or more selected from Pd/C with H₂, Raney nickel with H₂, or zinc powder with H₂. Such first reductants are known in the art, and are commercially available, or may be conventionally prepared by those skilled in the art.

In Step 6, preferably, the methylamination reagent used may be one or more selected from an aqueous formaldehyde solution, trioxymethylene, and paraformaldehyde. Such methylamination reagents are known in the art, and are commercially available, or may be conventionally prepared by those skilled in the art.

In the present invention, preferably, the reductive amination in Step 6 is actually a one-pot reaction of the deprotection and the reductive amination. In Step 6, by using such a first reductant and a methylamination reagent, both the removal of protecting groups R³ and R⁴ and the reductive dimethylation on the amino group can be achieved on Compound 10.

In Step 6, preferably, the organic solvent used may be an organic solvent independently selected from those solvents described above for Step 1.

In the present invention, more specifically, for example, Step 6 may be carried out as follows: Compound 10 is dissolved in an organic solvent, and stirring is started. Then, a methylamination reagent and a first reductant are added sequentially, and the reaction is carried out at room temperature for a period of time, such as 6 hours. Preferably, after the reductive amination reaction is completed, the resulting reaction mixture is subjected to post-treatments including extraction, washing, drying and column chromatography to obtain the compound of formula I. Specifically, for example, after the reductive amination reaction is completed, the resulting reaction mixture is suction filtered. Then, the organic phase is spin-dried, and an organic solvent and an aqueous solution such as an aqueous NaHCO₃ solution are added for layer separation. The organic layer is washed once with a saturated saline, dried over anhydrous sodium sulfate, and suction filtered. The filtrate is spin-dried and then is subjected to column chromatography to obtain the desired compound of formula I.

In the present invention, Compound 5 that is used as the starting compound may be prepared by using the commercially available Compound 2 as a raw material.

### Step 7

Compound 2 is reacted with Compound S5 under an alkaline condition to obtain Compound 3.

Preferably, Step 7 may be carried out as follows: Compound 2 as the starting compound is reacted with Compound S5 in an organic solvent in the presence of a base at a temperature of 40°C to 90°C to obtain Compound 3.

In the present invention, both the Compound 2 as a starting compound and the Compound S5 are commercially available, or may be synthesized or prepared according to the methods described in prior literatures.

In the present invention, the groups R¹ and R² in Compound S5 are as defined above for the Compound 5, and may be independently selected from H and C₁₋₁₀ alkyl.

In Step 7, preferably, the base used may be one or more selected from potassium carbonate, sodium carbonate, potassium phosphate, potassium bicarbonate and sodium bicarbonate.

In Step 7, preferably, the organic solvent used may be an organic solvent independently selected from those solvents described above for Step 1.

In the present invention, more specifically, for example, Step 7 may be carried out as follows: Compound 2, Compound S5 and an inorganic base are dissolved in an organic solvent, and stirring is started. Then, the water bath is heated to a temperature of 60°C to 80°C, and that temperature is kept overnight. Thereafter, water is added to terminate the reaction, and the mixture is cooled down to a temperature of 0°C to 10°C, at which the mixture is kept for 30 minutes, and then is suction filtered. The organic phase is spin-dried to obtain a concentrate. Then, the concentrate is recrystallized in a crystallization solvent by increasing the temperature to 40°C to 60°C. After that, the temperature is cooled down to 0°C, at which the mixture is kept for 30 minutes. The mixture is suction filtered, and the solid is dried obtain the Compound 3.

In Step 7, preferably, the crystallization solvent may be a mixed solvent, such as ethyl acetate/n-hexane, or dichloromethane/n-hexane.

### Step 8

Compound 3 is reduced with a second reductant to obtain Compound 4.

Preferably, Step 8 may be carried out as follows: the resultant Compound 3 is reduced in an organic solvent in the presence of a second reductant at a temperature of 0°C to 20°C to obtain Compound 4.

In Step 8, preferably, the second reductant may be one or more selected from sodium borohydride, potassium borohydride, borane, trimethylsilane, triethylsilane and tripropylsilane. Such second reductants are known in the art, and are commercially available, or may be conventionally prepared by those skilled in the art. By using such a second reductant, only the linking carbonyl between two benzene rings of Compound 3 can be reduced to a methylene group (-CH₂) in Step 8.

In Step 8, preferably, the organic solvent used may be an organic solvent independently selected from those solvents described above for Step 1.

In the present invention, more specifically, for example, Step 8 may be carried out as follows: Compound 3 is dissolved in an organic solvent, and stirring is started. Then, the mixture is cooled down to a temperature of 0°C to 10°C, and a second reductant is added. After that, the mixture is kept at that temperature overnight. Thereafter, water is added to terminate the reaction, and the mixture is separated into layers. The organic layer is washed twice with a saturated sodium bicarbonate aqueous solution, and washed twice with water. The organic phase is concentrated to dryness, and then is subjected to a column chromatography to obtain Compound 4.

### Step 9

Compound 4 is reduced with a third reductant to obtain Compound 5.

Preferably, Step 9 may be carried out as follows: the resultant Compound 4 is reduced in an organic solvent in the presence of a third reductant at a temperature of 0°C to 25°C to obtain Compound 5.

In Step 9, preferably, the third reductant used may be one or more selected from Pd/C/H₂, Raney nickel/H₂, iron/hydrochloric acid, or zinc/hydrochloric acid. Such third reductants are known in the art, and are commercially available, or may be conventionally prepared by those skilled in the art. By using such a third reductant, the nitro group (-NO₂) of Compound 4 can be reduced to an amino group (-NH₂) in Step 9.

In Step 9, preferably, the organic solvent used may be an organic solvent independently selected from those solvents described above for Step 1.

In the present invention, more specifically, for example, Step 9 may be carried out as follows: Compound 4 is dissolved in an organic solvent, and stirring is started. Then, a third reductant is added, and then the mixture is kept for 5 to 9 h. The mixture is suction filtered, and the filtrate is evaporated to dryness to obtain Compound 5.

The present invention also provides the intermediate compounds 3-10 below obtained through the method of the present invention: 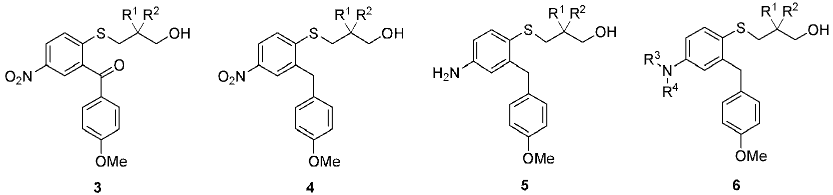 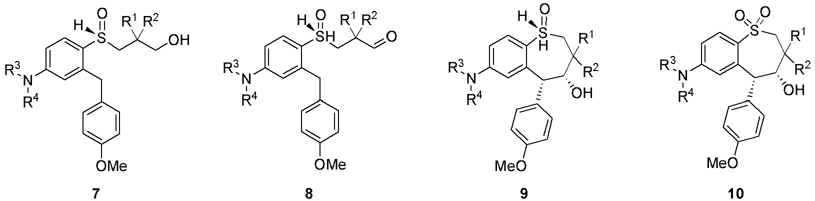 wherein R¹, R², R³ and R⁴ are as defined above. More specifically, in the method of the present invention, the following intermediate compounds are provided:

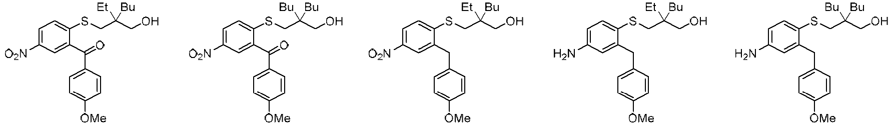

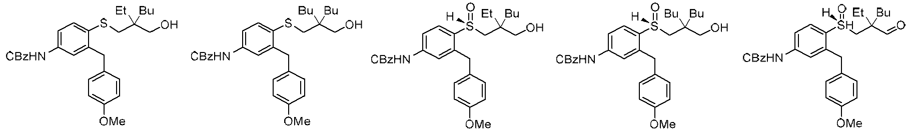

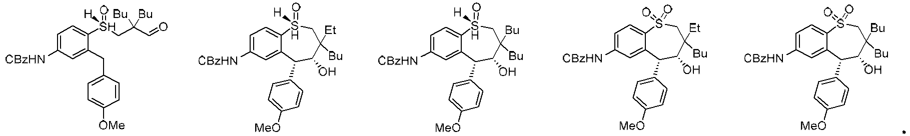

The compound of formula I and the above-mentioned intermediate compounds obtained through the method of the present invention can be used in preparing the drug Maralixibat. For example, the specific process may be as shown below in Reaction Scheme 4:

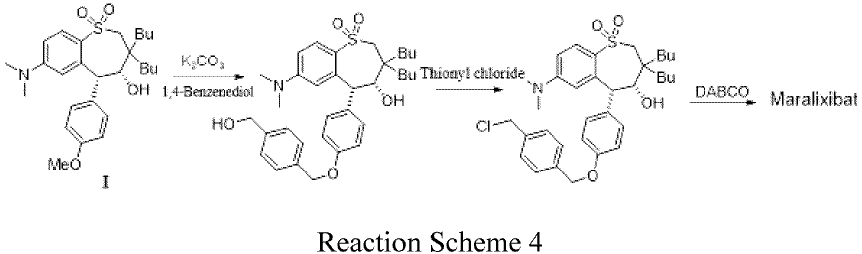

For example, regarding the specific reaction process for preparing the drug Maralixibat from the compound I of formula I, reference may be made to the disclosure of CN1284953A.

The raw materials used in the preparation method of the present invention are known and commercially available, or may be prepared by conventional methods or existing methods known to those skilled in the art, or may be easily prepared by similar methods to those known in the art. For example, the chiral ligand that can be used in the present invention may be purchased from DAICEL Chiral Technologies (Shanghai) Co., Ltd., and Compound S5 as a raw material may be prepared, for example, according to the contents recorded in the prior literature (Bioorg. Med. Chem. Lett. 24 (2014) 386-389).

The organic solvents, acids, bases, catalysts, protecting groups and so on used in the present invention are all commercial products, and are not particularly limited in use.

The abbreviations used in the above description and the examples below are as follows:
Cbz: benzyloxycarbonyl
CbzCl: benzyl chloroformate
DMP: dimethyl phthalate
NaHCO₃: sodium bicarbonate
H₂O₂: hydrogen peroxide
(Boc)₂O: di-tert-butyl dicarbonate
NaH: sodium hydride
KH: potassium hydride

Some preferred or particular compounds and reaction conditions that can be used in the present invention are described in detail by way of examples. However, the protection scope of the present invention is not limited thereto.

In addition, it should be noted that, in order to be better in accordance with the actual reaction operations and more convenient for reaction control, some parameters of the reaction conditions are presented as numerical ranges rather than specific numerical values in the following examples. As can be appreciated by those skilled in the art, such ranges mean that the corresponding reaction results can be achieved as long as the corresponding reaction conditions are controlled within the respective range (without being precisely a certain specific value).

### Example 1

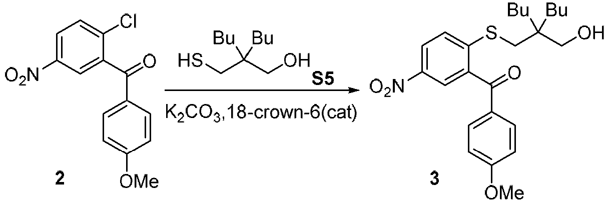

At room temperature, 70 g of Compound **2** (available from J&K Scientific), 36.1 g of potassium carbonate, 55.1 g of Compound **S5** (2-butyl-2-(mercaptomethyl)hexan-1-ol (which may be obtained according to the method described in the literature Bioorg. Med. Chem. Lett. 24 (2014) 386-389), and 300 mL of N,N-dimethylformamide were added into a reaction flask. The mixture was heated to a temperature of 70°C to 75°C, and then kept at that temperature overnight. After the reaction was completed, 900 mL of water was added dropwise, and a solid was precipitated out. The temperature was decreased to room temperature, then decreased to 0°C and kept for 30 minutes. The mixture was suction filtered. The filtrate was concentrated to dryness to obtain a concentrate. Then, 105 mL of ethyl acetate was added to the concentrate. The mixture was heated to a temperature of 50°C to 55°C, and then stirred until being dissolved to clearness. Hexane was added dropwise until a solid was precipitated out. The temperature was decreased to 0°C and kept for 30 minutes. The mixture was suction filtered, and the solid was oven dried to obtain 94.8 g of Compound **3** with a yield of 88%. ¹H NMR (400 MHz, chloroform-*d*) δ 8.23 (dd, *J* = 8.77, 2.53 Hz, 1H), 8.13 (d, *J=* 2.51 Hz, 1H), 7.76 (d, *J=* 8.88 Hz, 2H), 7.62 (d, *J=* 8.83 Hz, 1H), 6.95 (d, *J=* 8.87 Hz, 2H), 3.88 (s, 3H), 3.41 (d, *J=* 5.48 Hz, 2H), 2.99 (s, 2H), 1.30~1.12 (m, 12H), 0.85 (t, *J* = 7.07 Hz, 6H). ¹³C NMR (101 MHz, chloroform-*d*) δ 193.2, 164.4, 147.5, 144.3, 139.3, 132.6, 128.9, 128.3, 124.7, 123.3, 114.1, 65.8, 55.6, 41.4, 39.7, 32.8, 25.1, 23.3, 14.0.

### Example 2

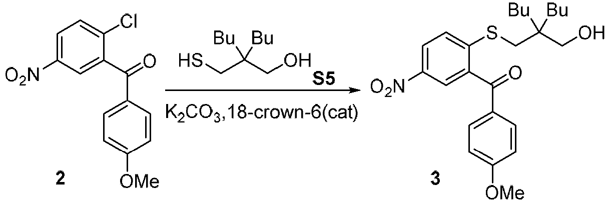

At room temperature, 70 g of Compound **2,** 54.6 g of potassium phosphate, 55.1 g of Compound **S5,** and 280 mL of tetrahydrofuran were added into a reaction flask. The mixture was heated to a temperature of 70°C, and kept at that temperature overnight under stirring. After the reaction was completed, the mixture was evaporated to dryness. 1000 mL of water was added into the resultant concentrate, which was slurried for 1 to 2 h. The mixture was suction filtered, and the filtrate was concentrated to dryness. Then, 105 mL of ethyl acetate was added to the resultant concentrate. The mixture was heated to a temperature of 50°C to 55°C, and stirred until being dissolved to clearness. Then, n-Hexane was added dropwise until a solid was precipitated out. Then, the temperature was decreased to room temperature, then decreased to 0°C and kept for 30 minutes. The mixture was suction filtered, and the solid was oven dried to obtain 92.9 g of Compound 3 with a yield of 85%.

### Example 3

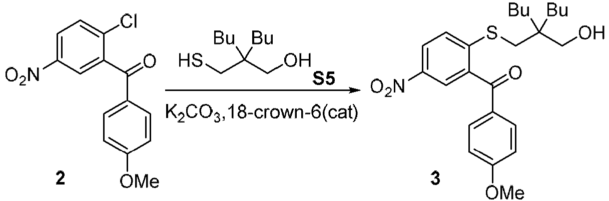

At room temperature, 70 g of Compound **2,** 27.7 g of sodium carbonate, 55.1 g of Compound **S5,** and 350 mL of methanol were added into a reaction flask. The mixture was heated to a temperature of 65-70°C, and kept at that temperature overnight. After the reaction was completed, the mixture was evaporated to dryness. Then, 850 mL of water was added into the resultant concentrate, which was slurried for 0.5 to 1 h. The temperature was decreased to 0°C and kept for 1-2 h. The mixture was suction filtered, and the filtrate was concentrated to dryness to obtain a concentrate. Then, 120 mL of dichloromethane was added to the concentrate. The mixture was heated to a temperature of 40°C, and stirred until being dissolved to clearness. Then, n-Heptane was added dropwise until a solid was precipitated out. The temperature was decreased to 0°C and kept for 60 minutes. The mixture was suction filtered, and the solid was oven dried to obtain 94.0 g of Compound **3** with a yield of 86%.

### Example 4

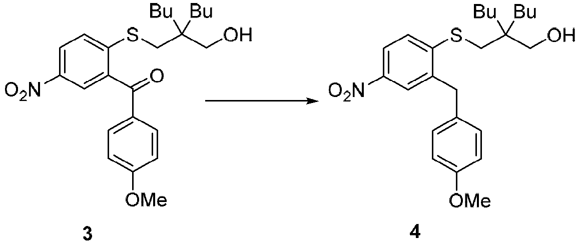

At room temperature, 140 g of Compound **3** and 900 g of dichloromethane were added into a reaction flask. The temperature was decreased to 0°C. Then, 195.2 g of trifluoromethanesulfonic acid and 114.2 g of triethylsilane were added in sequence. The mixture was kept at that temperature overnight with stirring. After the reaction was completed, ice water was slowly added to terminate the reaction, and the mixture was separated into layers. The organic layer was washed twice with a saturated sodium bicarbonate solution, and then washed twice with water. The organic phase was concentrated to dryness, and then subjected to a column chromatography to obtain 115.5 g of Compound **4** with a yield of 85%. ¹H NMR (400 MHz, chloroform-d) δ 8.02 (dd, *J* = 8.74, 2.58 Hz, 1H), 7.86 (d, *J* = 2.54 Hz, 1H), 7.38 (d, *J=* 8.78 Hz, 1H), 7.10 (m, *J* = 8.61 Hz, 2H), 6.85 (d, *J=* 8.66 Hz, 2H), 4.03 (s, 1H), 3.79 (s, 2H), 3.51 (d, *J* = 4.17 Hz, 1H), 3.00 (s, 1H), 1.43~1.18 (m, 14H), 0.91 (t, *J=* 7.13 Hz, 6H).

### Example 5

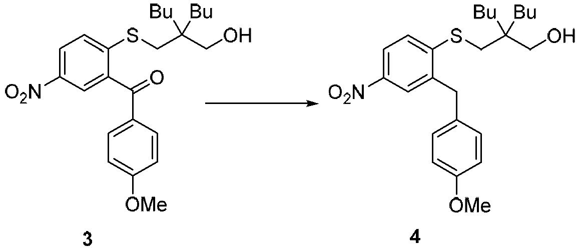

At room temperature, 140 g of Compound **3** and 1000 mL of 1,2-dichloroethane were added into a reaction flask. The temperature was decreased to 0°C. Then, 180.0 g of hydrochloric acid and 114.2 g of triethylsilane were added in sequence. The reaction mixture was stirred overnight. After the reaction was completed, a saturated sodium bicarbonate solution was slowly added to terminate the reaction, and the mixture was separated into layers. The organic layer was washed twice with saline, and then washed twice with water. The organic phase was concentrated to dryness, and then subjected to a column chromatography to obtain 111.3 g of Compound **4** with a yield of 83%.

### Example 6

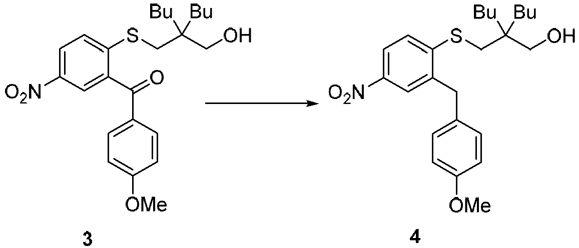

At room temperature, 140 g of Compound **3** and 940 mL of ethyl acetate were added into a reaction flask. The temperature was decreased to 0°C. Then, 240 g of glacial acetic acid and 114.2 g of triethylsilane were added in sequence. The reaction mixture was stirred overnight. Water was added, and the mixture was separated into layers. The organic layer was washed twice with water. The organic phase was concentrated to dryness, and then subjected to a column chromatography to obtain 114.0 g of Compound **4** with a yield of 84%.

### Example 7

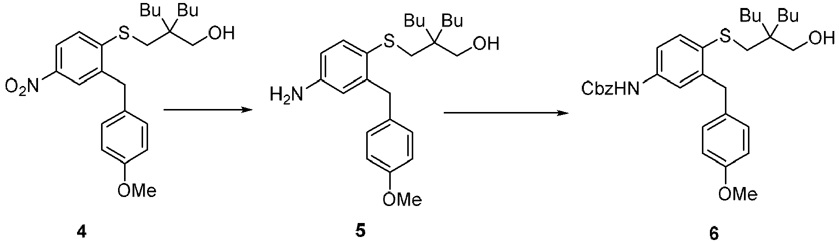

At room temperature, 50 g of Compound **4,** 500 mL of methanol, and 5.0 g of Raney nickel were added into an autoclave. After the atmosphere in the autoclave was replaced with hydrogen, the autoclave was sealed and kept at room temperature for 6 hours. After the reaction was completed, the mixture was suction filtered, and the filtrate was evaporated to dryness to obtain 86 g of Compound **5** with a crude product yield of 100%.

Next, 86 g of Compound **5,** 750 mL of tetrahydrofuran, and 42.5 g of micronized potassium carbonate were added into a reaction flask, and then stirring was started. The temperature was decreased to 5°C. Then, 38.6 g of CbzCl was slowly added dropwise. After that, the temperature was kept for 3-4 hours. After the reaction was completed, the solvent was evaporated, and 500 g of water and 500 g of ethyl acetate were added. The mixture was fully stirred, and then separated into layers. The aqueous phase was washed with ethyl acetate. The organic phases were combined and washed with 500 g of water. The organic phase was concentrated under vacuum to obtain 160 g of Compound **6** as an oil with a crude product yield of 100%.

### Example 8

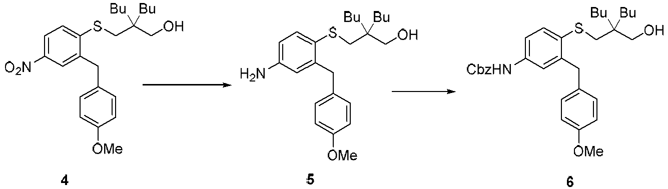

At room temperature, 60 g of Compound **4,** 360 mL of ethanol, and 4.8 g of Raney nickel were added into a reaction flask. After the atmosphere in the reaction flask was replaced with hydrogen, the reaction flask was kept at room temperature for 6 hours. After the reaction was completed, the mixture was suction filtered, and the filtrate was evaporated to dryness, and then evaporated twice with 150 mL of ethanol to obtain 103.2 g of Compound **5** with a crude product yield of 100%.

Next, 103.2 g of Compound **5,** 900 mL of acetonitrile, and 39.1 g of micronized sodium carbonate were added into a reaction flask, and then stirring was started. The temperature was decreased to 0-10°C. Then, 45.4 g of CbzCl was slowly added dropwise. After that, the temperature was kept for 3 hours. After the reaction was completed, 500 g of water was added, and the mixture was separated into layers. The organic phase was washed with 500 g of water, and evaporated to dryness to obtain 192 g of Compound **6** as an oil with a crude product yield of 100%.

### Example 9

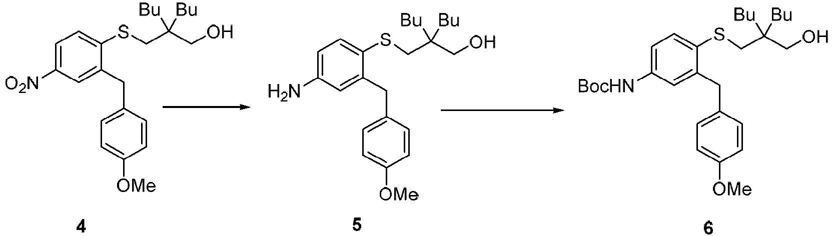

At room temperature, 40 g of Compound **4,** 400 mL of methanol, and 4.0 g of Raney nickel were added into a reaction flask 4.0 g under the protection of H₂, and then the temperature was kept for 6-7 hours. After the reaction was completed, the mixture was suction filtered, and the filtrate was evaporated to dryness to obtain 68.9 g of Compound **5** with a crude product yield of 100%.

Next, 68.8 g of Compound **5,** 600 mL of tetrahydrofuran, and 33.5 g of micronized potassium carbonate were added into a reaction flask, and then stirring was started. The temperature was decreased to 5°C. Then, 39.5 g of (Boc)₂O was slowly added dropwise. After that, the temperature was kept for 3 hours. After the reaction was completed, 400 g of water and 400 g of dichloromethane were added, and the mixture was separated into layers. The organic phase was washed once with 350 g of water, and evaporated to dryness to obtain 136.4 g of Compound **6** as an oil with a crude yield of 100%.

### Example 11

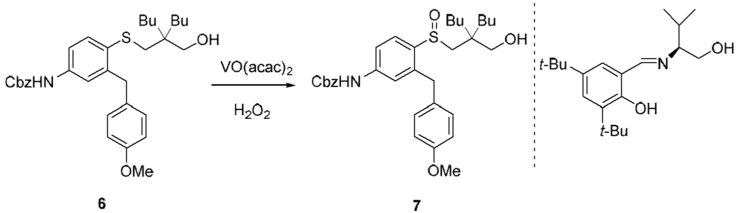

At room temperature, 26.5 mg (1 mol%) of vanadyl acetylacetonate and 40 mL of acetone were added, and dissolved to clearness. Then, 31.9 mg (1.04 mol%) of chiral ligand (specifically, as shown in the chemical formula at the right side of the dotted line in the above reaction formula) was further added. After stirring for 10 minutes, a solution of 5.5 g (10 mmol) Compound **6** in acetone (40 mL) was added. The temperature was decreased to -10°C. A solution of 30% hydrogen peroxide (1 mL) in acetone (30 mL) was slowly added dropwise to the above reaction solution with an injection pump at a flow rate of 3.1 mL/h, and the addition was completed within 10 h. After the reaction was completed, a dilute sodium thiosulfate solution was added and stirred to quench the reaction for 10 minutes. Then, the reaction solution was spin-dried, and about 200 mL of EA was added to dissolve the solid. The solution was washed with a saturated saline. The EA phase was dried by adding anhydrous sodium sulfate. The desiccant was filtered off, and the filtrate was spin-dried to obtain 5.65 g of solid with a crude product yield of 99.9%. A sample of the crude product was taken and subjected to a chiral HPLC detection, and the ee value was 89.1%. Then, 15 mL of isopropanol and 0.75 mL of water were added to the crude product, and the mixture was heated to 80°C until the solid was completely dissolved. The temperature was slowly decreased to 40°C and kept for 1 to 2 h. After that, the heating was turned off, and the temperature was decreased to room temperature. Then, a solid was gradually precipitated out. The mixture was suction filtered to obtain 5.6 g of Compound **7** with a yield of 99% and an ee value of 99.5%. ¹H NMR (400 MHz, chloroform-*d*) δ 7.96 (d, *J =* 8.60 Hz, 1H), 7.55 (dd, *J =* 8.62, 2.25 Hz, 1H), 7.44~7.36 (m, 6H), 7.02 (d, *J* = 8.20 Hz, 2H), 6.84 (d, *J* = 8.54 Hz, 2H), 5.24 (s, 2H), 4.04 (s, 2H), 3.80 (s, 3H), 3.69 (d, *J=* 12.21 Hz, 1H), 3.46 (d, *J=* 12.22 Hz, 1H), 2.78 (d, *J =* 13.81 Hz, 1H), 2.24 (d, *J =* 13.83 Hz, 1H), 1.61 (dd, *J* = 12.81, 9.70 Hz, 1H), 1.38~1.09 (m, 11H), 0.95 (t, *J* = 7.20 Hz, 3H), 0.87 (t, *J=* 7.30 Hz, 3H). ¹³C NMR (101 MHz, chloroform-*d*) δ 158.4, 153.1, 140.9, 139.0, 136.7, 135.8, 130.8, 129.6, 128.7, 128.6, 128.4, 126.0, 120.3, 118.1, 114.3, 68.0, 67.4, 55.3, 42.2, 37.1, 35.6, 32.5, 25.1, 25.0, 23.5, 23.3, 14.2, 14.0.

### Example 12

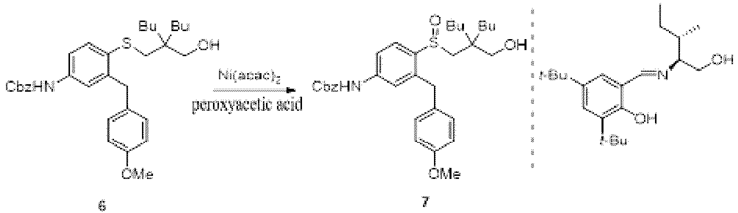

At room temperature, 25.6 mg (1 mol%) of nickel acetylacetonate (Ni(acac)₂), 33.3 mg (1.04 mol%) of chiral ligand (specifically, as shown in the chemical formula at the right side of the dotted line in the above reaction formula), 5.5 g (10 mmol) of Compound **6**, and 40 mL of acetonitrile were added, and then the mixture was dissolved to clearness. The temperature was decreased to -10°C. Then, a solution of 0.76 g peroxyacetic acid in acetonitrile (30 mL) was slowly added, and the temperature was kept for 3 hours. After the reaction was completed, the mixture was suction filtered, and the mother liquor was spin-dried. Then, 200 mL of ethyl acetate was added to dissolve the solid. The solution was washed with a saturated saline. The organic phase was spin-dried to obtain 5.65 g of solid with a crude product yield of 99.9%. A sample of the solid was taken and subjected to a chiral HPLC, and the ee value was 89.1%. Then, 50 mL of methanol and 60 mL of water were added to the crude product, and the mixture was heated to 50°C until the solid was completely dissolved. The temperature was slowly decreased to room temperature and kept for 2 h. After that, the heating was turned off, and the temperature was decreased to room temperature. Then, a solid was gradually precipitated out. The mixture was suction filtered to obtain 5.6 g of Compound **7** with a yield of 99% and an ee value of 98.9%.

### Example 13

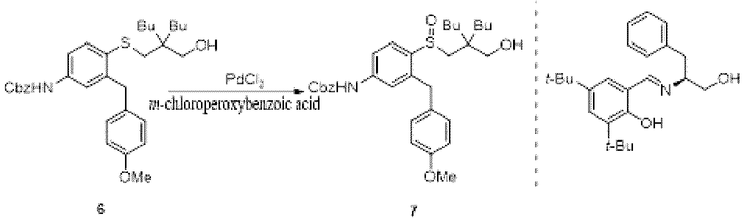

At room temperature, 17.7 mg (1 mol%) of palladium dichloride and 40 mL of isopropanol were added, and the mixture was dissolved to clearness. Then, 36.7 mg (1.04 mol%) of chiral ligand (specifically, as shown in the chemical formula at the right side of the dotted line in the above reaction formula) was further added. After stirring for 10 minutes, a solution of 5.5 g (10 mmol) Compound 6 in isopropanol (10 mL) was added. The temperature was decreased to -10°C. A solution of 1.72 g *m*-chloroperoxybenzoic acid in isopropanol (10 mL) was slowly added, and the reaction was kept at that temperature for 2 to 3 h. The reaction mixture was suction filtered and concentrated to dryness, and then 15 mL of isopropanol and 0.75 mL of water was added. The mixture was heated to 80°C until the solid was completely dissolved. The temperature was slowly decreased to 40°C and kept for 2 h. After that, the heating was turned off, and the temperature was decreased to room temperature. Then, a solid was gradually precipitated out. The mixture was suction filtered to obtain 5.6 g of Compound **7** with a yield of 99% and an ee value of 99.8%.

### Example 14

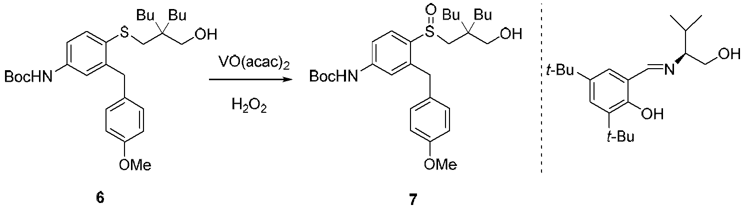

At room temperature, 26.5 mg (1 mol%) of vanadyl acetylacetonate and 40 mL of acetone were added, and the mixture was dissolved to clearness. Then, 31.9 mg (1.04 mol%) of chiral ligand (specifically, as shown in the chemical formula at the right side of the dotted line in the above reaction formula) was further added. After stirring for 10 minutes, a solution of 5.15 g (10 mmol) of Compound **6** in acetone (40 mL) was added. The temperature was decreased to -10°C. A solution of 1.72 g *m*-chloroperoxybenzoic acid in acetone (10 mL) was slowly added, and the reaction was kept at that temperature for 3 h. The reaction mixture was suction filtered and concentrated to dryness, and a crude product was obtained. Then, 12 mL of isopropanol and 1.1 mL of water were added to the crude product, and the mixture was heated to 80°C until the solid was completely dissolved. The temperature was slowly decreased to 40°C and kept for 2 h. After that, the heating was turned off, and the temperature was decreased to room temperature. Then, a solid was gradually precipitated out. The mixture was suction filtered to obtain 5.2 g of Compound **7** with a yield of 98% and an ee value of 99.1%.

### Example 15

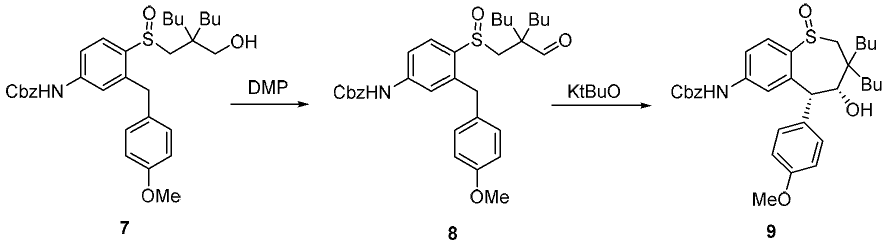

At room temperature, 2.43 g of Compound **7** (4.3 mmol) and 25 mL of dichloromethane were added. The mixture was cooled down to 15°C, and 2.19 g (11.3 mmol) of dimethyl phthalate was added in batches, then the mixture was naturally warmed up with stirring for 1 h. After the reaction was completed, diatomite was added for filtration. The filtrate was spin-dried, and 75 mL of methyl tert-butyl ether was added. Then, 25 mL of saturated sodium bicarbonate solution was added to adjust the pH to 7. The mixture was stirred for 3 hours at room temperature, and then a solid was precipitated out. The reaction mixture was filtered, and the filter cake was washed several times with methyl tert-butyl ether. The filtrates were combined, and a saturated saline was added. The organic layer was dried by adding anhydrous sodium sulfate, and then was suction filtered. The filtrate was spin-dried to obtain 2.43 g of the product Compound **8** with a yield of 100%, which was directly used in the next reaction. ¹H NMR (400 MHz, chloroform-*d*) δ 9.46 (s, 1H), 7.92 (d, *J* = 8.66 Hz, 1H), 7.51 (dd, *J=* 8.61, 2.24 Hz, 1H), 7.37 (dd, *J=* 3.59, 1.29 Hz, 4H), 7.25 (d, *J* = 4.15 Hz, 1H), 6.99 (d, *J=* 8.41 Hz, 3H), 6.80 (d, *J=* 8.63 Hz, 2H), 5.18 (s, 2H), 4.01 (q, *J=* 15.74 Hz, 2H), 3.75 (s, 3H), 2.90 (d, *J=* 13.96 Hz, 1H), 2.46 (d, *J=* 14.02 Hz, 1H), 1.88 (td, *J=* 13.23, 12.14, 3.99 Hz, 1H), 1.67~1.56 (m, 4H), 1.36~1.18 (m, 5H), 1.10~1.01 (m, 3H), 0.87 (dt, *J=* 20.31, 7.19 Hz, 6H). ¹³C NMR (101 MHz, chloroform-*d*) δ 204.0, 158.4, 153.0, 140.8, 139.2, 137.7, 135.7, 131.0, 129.7, 128.6, 128.4, 128.3, 125.9, 120.1, 118.0, 114.2, 67.3, 60.9, 55.2, 51.9, 36.9, 33.0, 32.0, 25.7, 25.5, 23.1, 23.1, 13.8, 13.7.

Next, 1.13 g of Compound **8** (2 mmol) and 30 mL of anhydrous tetrahydrofuran were added under a nitrogen atmosphere. Then, 5.0 mL of 1 mol/L potassium tert-butoxide solution was added dropwise at room temperature. After completion of the dropwise addition, the mixture was heated to 40°C and then further stirred for 10 h. After the reaction was completed, the temperature was decreased to -15°C, and 20 mL of water was added dropwise to quench the reaction, then 0.5 mL of hydrochloric acid was further added dropwise to adjust the pH to 3. After the adjustment, the mixture was separated into layers. The aqueous layer was extracted with EA, and the organic layers were combined. The organic layer was washed sequentially with a saturated NaHCO₃ aqueous solution and a saturated saline, and then spin-dried to obtain 1.14 g of the crude product of Compound **9** with a crude yield of 100%, which was directly used in the next reaction. ¹H NMR (400 MHz, chloroform-*d*) δ 7.72 (s, 2H), 7.36 (d, *J =* 8.61 Hz, 2H), 7.30 (s, 5H), 6.90 (d, *J=* 8.74 Hz, 2H), 6.57 (s, 1H), 5.10 (s, 2H), 4.44 (s, 1H), 4.10 (q, *J=* 7.15 Hz, 1H), 3.94 (s, 1H), 3.79 (s, 3H), 2.95 (d, *J* = 12.95 Hz, 1H), 2.83 (dd, *J* = 12.97, 2.63 Hz, 1H), 2.02 (s, 2H), 1.87 (td, *J* = 13.20, 3.68 Hz, 1H), 1.64~1.57 (m, 1H), 1.47~1.30 (m, 7H), 0.90 (dt, *J* = 17.29, 7.12 Hz, 6H). ¹³C NMR (101 MHz, chloroform-*d*) δ 158.7, 153.0, 139.9, 136.2, 135.8, 134.0, 129.8, 128.5, 128.3, 128.2, 123.8, 121.0, 117.8, 114.3, 74.5, 67.1, 60.3, 59.6, 55.2, 46.7, 44.9, 35.4, 31.8, 25.0, 24.3, 23.3, 23.2, 13.9.

### Example 16

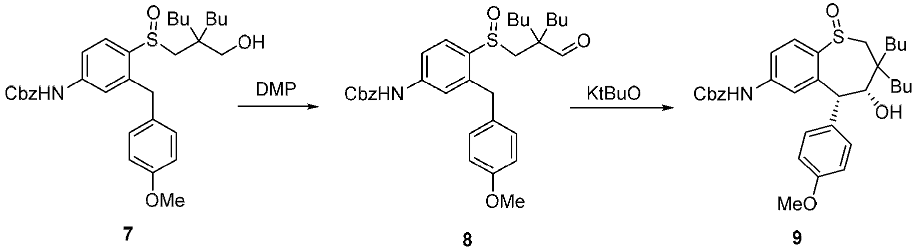

At room temperature, 2.69 g of Compound **7** (5.0 mmol) and 25 mL of dichloromethane were added. The mixture was cooled down to 10°C, and 2.41 g (12.5 mmol) of dimethyl phthalate was added in batches, then the mixture was naturally warmed up with stirring for 1 h. After the reaction was completed, diatomite was added for filtration. The filtrate was spin-dried, and 45 mL of dichloromethane was added. Then, 25 mL of saturated sodium bicarbonate aqueous solution was added to adjust the pH to 8. The mixture was stirred for 5 hours at room temperature, and then a solid was precipitated out. The reaction mixture was filtered, and the filtrate was washed with a saturated saline. The organic layer was dried by adding anhydrous sodium sulfate, and then suction filtered. The filtrate was spin-dried to obtain 2.67 g of the product Compound **8** with a yield of 100%, which was directly used in next reaction.

Next, 1.07 g of Compound **8** (2 mmol) and 25 mL of anhydrous acetonitrile were added under a nitrogen atmosphere. Then, 5.0 mL of 1 mol/L potassium tert-butoxide solution was added dropwise at room temperature. After completion of the dropwise addition, the mixture was heated to 45°C and then further stirred for 12 h. After the reaction was completed, the temperature was decreased to -15°C, and 20 mL of water was added dropwise to quench the reaction, then glacial acetic acid was added dropwise to adjust the pH to 5. After the adjustment, the mixture was separated into layers. The aqueous layer was extracted with EA, and the organic layers were combined. The organic layer was washed sequentially with a saturated NaHCO₃ aqueous solution and a saturated saline, and then spin-dried to obtain 1.07 g of the crude product of Compound **9** with a crude yield of 100%, which was directly used in the next reaction.

### Example 17

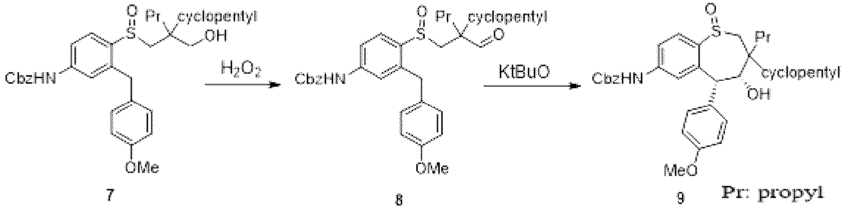

At room temperature, 2.89 g of Compound **7** (5.0 mmol) and 30 mL of dichloromethane were added. The mixture was cooled down to 10°C, and 10 mL of aqueous hydrogen peroxide solution was added in batches, then the mixture was naturally warmed up with stirring for 1 h. After the reaction was completed, the organic phase was washed twice with a saturated saline. The filtrates were combined and spin-dried to obtain 2.89 g of the product Compound **8** with a yield of 100%, which was directly used in the next reaction.

Next, 1.15 g of Compound **8** (2 mmol) and 30 mL of anhydrous dichloromethane were added under a nitrogen atmosphere. 5.0 mL of 1 mol/L sodium tert-butoxide solution (2.5 mmol) was added dropwise at room temperature. After completion of the dropwise addition, the mixture was heated to 60°C and then further stirred for 12 h. After the reaction was completed, the temperature was decreased to 0°C, and methanesulfonic acid was added dropwise to adjust the pH to 3. After the adjustment, the mixture was separated into layers. The organic layer was washed sequentially twice with a saturated NaHCO₃ aqueous solution, and then spin-dried to obtain 1.16 g of the crude product of Compound **9** with a crude yield of 100%, which was directly used in the next reaction.

### Example 18

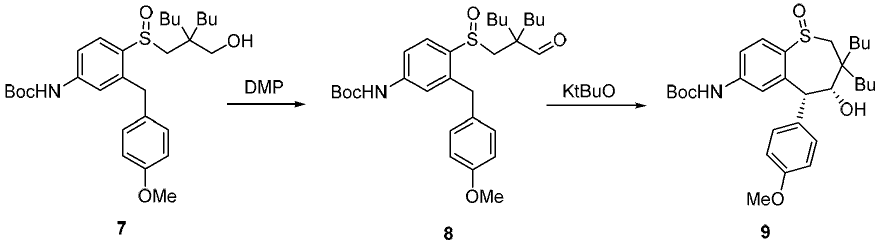

At room temperature, 2.28 g of Compound **7** (4.3 mmol) and 20 mL of acetone were added. The mixture was cooled down to 15°C, and 2.54 g (6.0 mmol) of dimethyl phthalate was added in batches, then the mixture was naturally warmed up with stirring for 1 h. After the reaction was completed, diatomite was added for filtration to obtain a filtrate, and a saturated NaHCO₃ aqueous solution was added thereto to adjust the pH to 7. The mixture was stirred for 3 hours at room temperature, and then a solid was precipitated out. The reaction mixture was filtered, and the filter cake was washed several times with acetone. The filtrates were combined, and a saturated saline was added. The mixture was separated into layers. The organic phase was spin-dried to obtain 2.43 g of the product Compound **8** with a yield of 100%, which was directly used in the next reaction.

Next, 1.06 g of Compound **8** (2 mmol) and 30 mL of acetone were added under a nitrogen atmosphere. Then, 5.0 mL of 1 mol/L potassium tert-butoxide solution (2.5 mmol) was added dropwise at room temperature. After completion of the dropwise addition, the mixture was heated to 40°C and then further stirred for 10 h. After the reaction was completed, the temperature was decreased to 0°C, and an aqueous citric acid solution was added dropwise to adjust the pH to 4. After the adjustment, the mixture was separated into layers. The aqueous layer was extracted with EA, and the organic layers were combined. The organic layer was washed with a saturated saline, and then spin-dried to obtain 1.06 g of the crude product of Compound **9** with a crude yield of 100%, which was directly used in the next reaction.

### Example 19

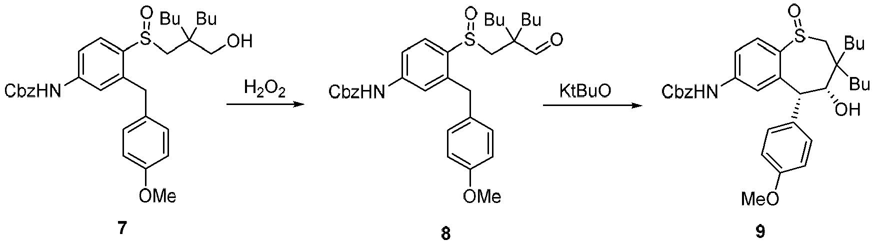

At room temperature, 2.43 g of Compound **7** (4.3 mmol) and 25 mL of acetonitrile were added. The mixture was cooled down to 15°C, and 10 mL of aqueous hydrogen peroxide solution was added in batches, then the mixture was naturally warmed up with stirring for 1 h. After the reaction was completed, the reaction mixture was concentrated to dryness, and ethyl acetate was added to dissolve to clearness. The organic phase was washed twice with a saturated saline. The filtrates were combined, and then spin-dried to obtain 2.43 g of the product Compound **8** with a yield of 100%, which was directly used in the next reaction.

Next, 1.13 g of Compound **8** (2 mmol) and 30 mL of acetonitrile were added under a nitrogen atmosphere. Then, 5.0 mL of 1 mol/L potassium tert-butoxide solution (2.5 mmol) was added dropwise at room temperature. After completion of the dropwise addition, the mixture was heated to 40°C and then further stirred for 10 h. After the reaction was completed, the temperature was decreased to -15°C, and 20 mL of water was added dropwise to quench the reaction, then 0.5 mL of hydrochloric acid was further added dropwise to adjust the pH to 3. After the adjustment, the mixture was separated into layers. The aqueous layer was extracted with EA, and the organic layers were combined. The organic layer was washed sequentially with a saturated NaHCO₃ aqueous solution and a saturated saline, and then spin-dried to obtain 1.14 g of the crude product of Compound **9** with a crude yield of 100%, which was directly used in the next reaction.

### Example 20

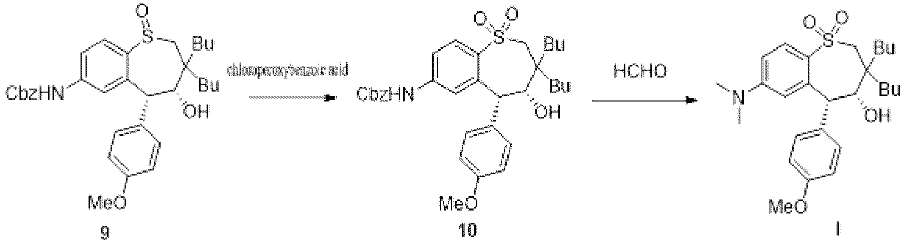

At room temperature, 1.14 g of Compound **9** and 20 mL of methanol were added, and then dissolved to clearness. Then, the temperature was decreased to 0°C, and 508 mg of chloroperoxybenzoic acid was added. After the addition was completed, the mixture was warmed up to room temperature, and then kept at that temperature for 1 h. After the reaction was completed, methanol was spin-dried, and 20 mL of ethyl acetate was added. Then, a saturated NaHCO₃ aqueous solution was added to adjust the pH to 7. The mixture was extracted and separated into layers. The organic layer was washed with a saturated saline to obtain an organic phase. The organic phase was spin-dried, and then subjected to a column chromatography to obtain 1.06 g of the product Compound **10** with a three-step yield of 92.1%, an ee value of 99.9%, and a dr value of > 20:1.

Next, 647 mg (1.12 mmol) of Compound **10** and 10 mL of acetonitrile were added, and stirred to dissolve to clearness. Then, 453 mg of 37% formaldehyde aqueous solution, 64 mg of Pd/C, and 26 mg of concentrated sulfuric acid were sequentially added. The reaction system was supplied with hydrogen to a pressure of 5 atm, and reacted at room temperature for 6 hours. Then, Pd/C was filtered out and recycled. Then, 60 mL of ethyl acetate and a NaHCO₃ aqueous solution were added. The mixture was separated into layers. The organic layer was washed once with a saturated NaCl aqueous solution. The filtrate was spin-dried, and then subjected to a column chromatography to obtain 725.6 mg of the compound of formula I with a yield of 98.5%, an ee value of 99.9%, and a dr value of > 20:1. ¹H NMR (400 MHz, chloroform-*d*) δ 7.93 (d, *J=* 8.83 Hz, 1H), 7.46 (d, *J=* 8.15 Hz, 2H), 6.97 (d, *J=* 8.59 Hz, 2H), 6.55 (dd, *J=* 8.89, 2.61 Hz, 1H), 6.02 (d, *J=* 2.57 Hz, 1H), 5.52 (s, 1H), 4.15 (d, *J* = 6.07 Hz, 1H), 3.88 (s, 3H), 3.18 (d, *J =* 15.18 Hz, 1H), 3.03 (d, *J* = 15.12 Hz, 1H), 2.85 (s, 6H), 2.25 (td, *J=* 14.45, 13.71, 3.69 Hz, 1H), 1.66 (td, *J* = 13.57, 12.86, 4.06 Hz, 2H), 1.54~1.38 (m, 2H), 1.33~1.11 (m, 7H), 0.94 (q, *J* = 6.88 Hz, 6H). ¹³C NMR (101 MHz, chloroform-*d*) δ 158.5, 153.1, 139.8, 135.1, 130.1, 129.3, 126.0, 114.6, 108.7, 57.5, 55.4, 46.3, 44.2, 39.8, 35.4, 29.8, 25.0, 24.5, 23.3, 14.2, 14.1.

### Example 21

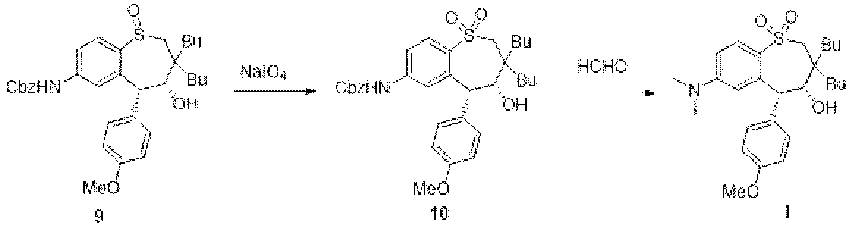

At room temperature, 1.14 g of Compound **9** and 20 mL of 1,2-dichloroethane were added and dissolved to clearness. Then, the temperature was decreased to 0°C, and 486 mg of sodium periodate was added. After the addition was completed, the mixture was warmed up to room temperature and kept at that temperature for 1 h. After the reaction was completed, the reaction mixture was suction filtered, and then evaporated to dryness to obtain a concentrate. The concentrate was subjected to a column chromatography to obtain 1.15 g of Compound **10** with a three-step yield of 90%, an ee value of 99.7%, and a dr value of > 20:1.

Next, 647 mg (1.12 mmol) of Compound **10** and 10 mL of ethanol were added, and stirred to dissolve to clearness. Then, 410 mg of paraformaldehyde, 64 mg of Pd/C, and 45 mg of concentrated hydrochloric acid were sequentially added. The reaction system was supplied with hydrogen to a pressure of 5 atm, and reacted at room temperature for 6 hours. Then, Pd/C was filtered out and recycled. Then, Ethanol was spin-dried, and EA and a NaHCO₃ aqueous solution were added. The mixture was separated into layers. The organic layer was washed once with a saturated NaCl aqueous solution, dried over anhydrous sodium sulfate, and suction filtered. The filtrate was spin-dried, and then subjected to a column chromatography to obtain 722 mg of the compound of formula I with a yield of 98%, an ee value of 99.7%, and a dr value of > 20:1.

### Example 22

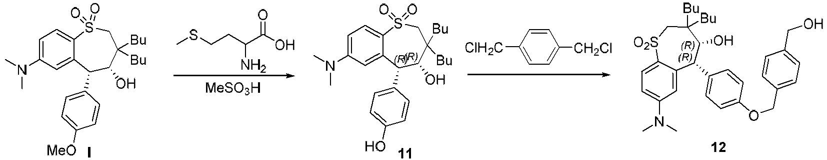

At room temperature, 13 g of the compound of formula I, 5.9 g of L-methionine, and 76 mL of methylsulfonic acid were added into a reaction flask under the protection of nitrogen. The temperature was increased to 75°C, and the reaction was kept at that temperature for 8 hours. After the reaction was completed, 100 g of water and 180 g of ethyl acetate were added for extraction. The organic phase was washed sequentially with 100 mL of saturated sodium bicarbonate aqueous solution and 100 g×2 of water, and then concentrated under vacuum to obtain a concentrate. The concentrate was subjected to a column chromatography to obtain 12.1 g of white Compound **11** with a yield of 96%, an ee value of 99.9%, and a dr value of > 20:1.

Next, 12 g of Compound **11,** 2.0 g of 50% NaOH aqueous solution and 80 g of N,N-dimethylacetamide were added into a reaction flask and stirred under the protection of nitrogen. Then, the temperature was increased to 50°C. After 30 minutes of reaction, a mixed solution composed of 4 g of 4-(chloromethyl)-benzyl alcohol and 10 mL of N,N-dimethylacetamide was further added. The mixture was reacted at 50°C for 24 hours. After the reaction was completed, 120 g of toluene and 70 g of water were added, and stirred at 50°C for 10 minutes. The mixture was separated into layers. The organic phase was washed with 100 g×2 saturated NaCl aqueous solution, concentrated, and then subjected to a column chromatography to obtain 15 g of white Compound **12** with a yield of 99.0%, an ee value of 99.9%, and a dr value of > 20:1.

### Example 23

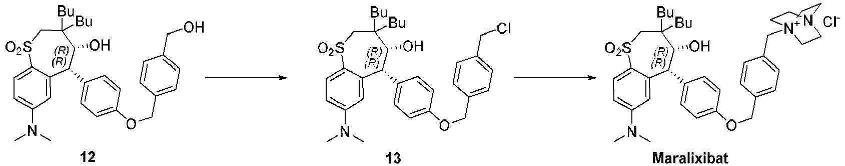

At room temperature, 15 g of Compound **12** and 80 g of toluene were added into a reaction flask under the protection of nitrogen, and dissolved to clearness. Then, 3.3 g of thionyl chloride was added dropwise, and the mixture was reacted at 35°C for 30 minutes. After the reaction was completed, 250 mL of NaOH aqueous solution with a concentration of 0.25 mol/ L was added. The mixture was separated into layers. The organic phase was washed twice with 100 mL×2 NaCl solution, and evaporated to dryness to obtain a concentrate. The concentrate was subjected to a column chromatography to obtain **14.78** g of white Compound 13 with a yield of 95%, an ee value of 99.9%, and a dr value of > 20:1.

Next, 13 g of Compound **13,** 2.68 g of triethylenediamine, 280 mL of acetonitrile were added into a reaction flask under the protection of nitrogen. The mixture was heated to 35°C and reacted for 2 hours. After the reaction was completed, the reaction mixture was suction filtered to obtain 16.1 g of white solid. The white solid was recrystallized in methanol/diethyl ether to obtain 14.1 g of the drug compound Maralixibat as a white solid with a yield of 84%, an ee value of 99.9%, and a dr value of > 20:1 (for the characterization data for the drug compound Maralixibat, reference may be made to Embodiment 10 of CN1284953A).

The above description is only some preferred embodiments and/or examples of the present invention. It should be noted that many modifications and variations can be made by one of ordinary skill in the art without departing from the principle of the present invention. These modifications and variations also fall within the protection scope of the present invention.

## Claims

1. A method for preparing a compound of formula I, comprising: 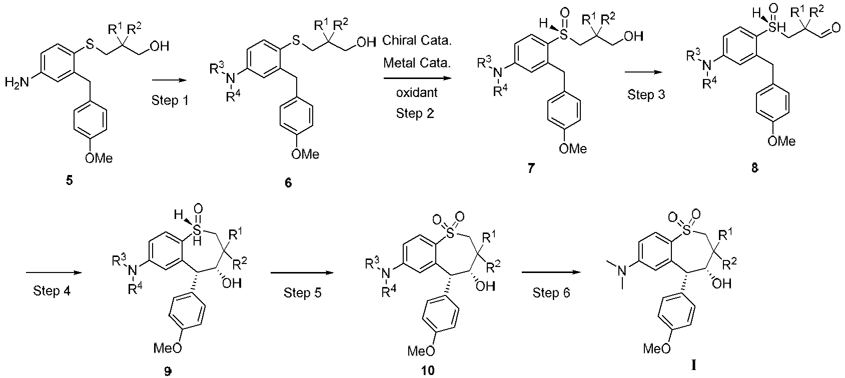
Step 1: reacting Compound 5 with a protecting reagent bearing amino-protecting groups R³ and R⁴ to obtain Compound 6;
Step 2: oxidizing the Compound 6 with a first oxidant in the presence of a chiral ligand and a metal catalyst to obtain Compound 7;
Step 3: reacting the Compound 7 with a second oxidant to obtain Compound 8;
Step 4: cyclizing the Compound 8 under an alkaline condition to obtain Compound 9;
Step 5: reacting the Compound 9 with a third oxidant to obtain Compound 10; and
Step 6: reacting the Compound 10 with a methylamination reagent in the presence of a first reductant to obtain the compound of formula I,
wherein R¹ and R² are independently selected from H and C₁₋₁₀ alkyl, R³ and R⁴ are amino-protecting groups independently selected from H, benzyl, 4-methoxybenzyl, benzylformyl, tert-butoxycarbonyl, formyl, acetyl, trifluoroacetyl and 9-fluorenylmethoxycarbonyl, and both R³and R⁴ are not simultaneously H.

2. The method of claim 1, **characterized in that** R¹ and R² are independently selected from H, methyl, ethyl, n-propyl, isopropyl, n-butyl, isobutyl, n-pentyl, isopentyl, n-hexyl, n-heptyl, n-octyl, n-nonyl and n-decyl.

3. The method of claim 1, **characterized in that**, R¹ and R² are independently selected from H, methyl, ethyl, n-propyl and n-butyl.

4. The method of claim 1, **characterized in that** the chiral ligand used in Step 2 is one or more selected from

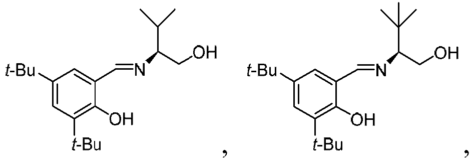

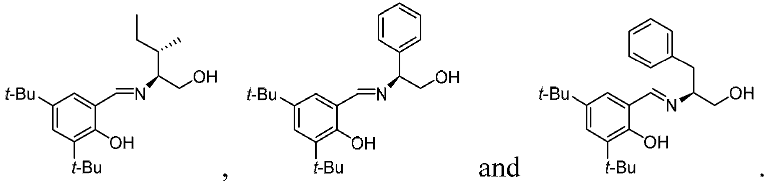

5. The method of claim 1, **characterized in that** the metal catalyst used in Step 2 is one or more selected from PdCl₂, NiCl₂, Pd(OH)₂, Ni(acac)₂, RhCl₂ and CrCl₂.

6. The method of claim 1, **characterized in that** the first oxidant used in Step 2 is one or more selected from H₂O₂, 3-phenyl-2-phenylsulfonyl-1,2-oxaziridine, and benzoyl peroxide.

7. The method of claim 1, **characterized in that** the second oxidant used in Step 3 is one or more selected from potassium permanganate, manganese dioxide, and sodium hypochlorite.

8. The method of claim 1, **characterized in that** a base used for the alkaline condition in Step 4 is one or more selected from sodium methoxide, sodium ethoxide, sodium tert-butoxide, potassium tert-butoxide, sodium hydroxide, potassium hydroxide, cesium hydroxide, sodium carbonate, potassium carbonate, potassium bicarbonate, NaH, KH, lithium diisopropylamide, lithium hexamethyldisilamide, and sodium hexamethyldisilamide.

9. The method of claim 1, **characterized in that** the third oxidant used in Step 5 is one or more selected from peroxyacetic acid, sodium periodate, and m-chloroperoxybenzoic acid.

10. The method of claim 1, **characterized in that** the first reductant used in Step 6 is one or more selected from Pd/C with H₂, Raney nickel with H₂, or zinc powder with H₂.

11. The method of claim 1, **characterized in that** the methylamination reagent used in Step 6 is one or more selected from formaldehyde, trioxymethylene, and paraformaldehyde.

12. The method of claim 1, **characterized in that** the Compound 5 used in Step 1 is prepared by a method comprising steps of: 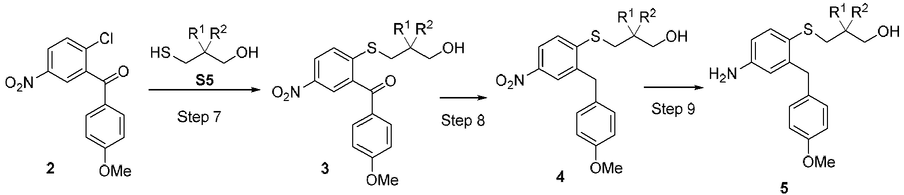
Step 7: reacting Compound 2 with Compound S5 under an alkaline condition to obtain Compound 3;
Step 8: reducing the Compound 3 with a second reductant to obtain Compound 4; and
Step 9: reducing the Compound 4 with a third reductant to obtain the Compound 5;
wherein R¹ and R² are as defined above.

13. The method of claim 12, **characterized in that** a base used for the alkaline condition in Step 7 is one or more selected from potassium carbonate, sodium carbonate, potassium phosphate, potassium bicarbonate, and sodium bicarbonate.

14. The method of claim 12, **characterized in that** the second reductant used in Step 8 is one or more selected from sodium borohydride, potassium borohydride, borane, trimethylsilane, triethylsilane, and tripropylsilane.

15. The method of claim 12, **characterized in that** the third reductant used in Step 9 is one or more selected from Pd/C/H₂, Raney nickel/H₂, iron/hydrochloric acid, or zinc/hydrochloric acid.

16. Intermediate compounds as shown below for preparing the drug Maralixibat: 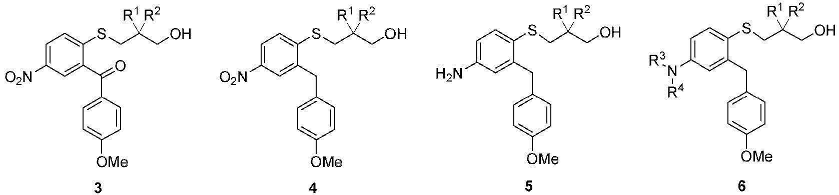 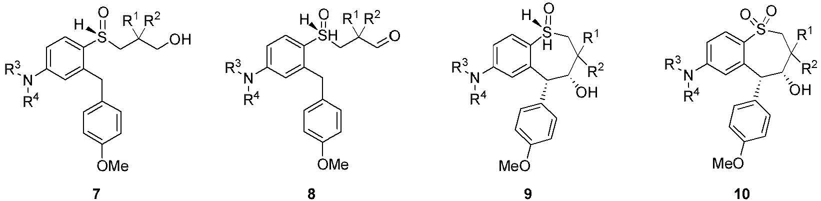 wherein R¹, R², R³ and R⁴ are as defined above.

17. The intermediate compounds of claim 16, **characterized in that** the intermediate compounds are selected from: 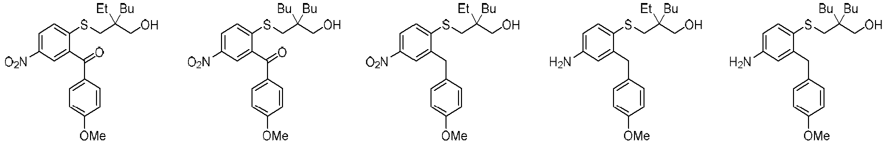 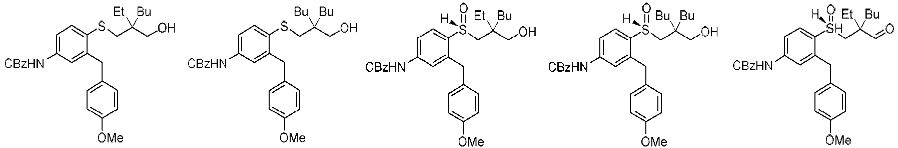 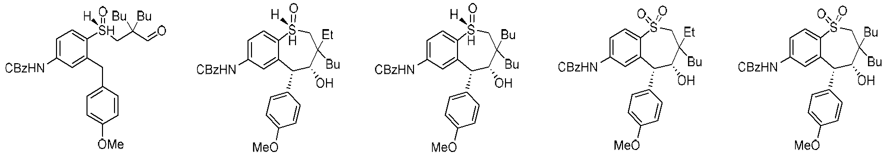 wherein Me represents methyl, Et represents ethyl, Bu represents butyl, and CBz represents benzyloxycarbonyl.

18. Use of the compound of formula I according to any one of claims 1-15 or the intermediate compounds according to claim 16 or 17 in preparing the drug Maralixibat.
